Europäisches Patentamt

European Patent Office

Office européen des brevets

(19)

(11) Veröffentlichungsnummer: **0 291 921 B1**

(12) # EUROPÄISCHE PATENTSCHRIFT

(45) Veröffentlichungstag der Patentschrift: **02.10.91**

(51) Int. Cl.⁵: **A61M  16/00, B01D 63/02**

(21) Anmeldenummer: **88107859.6**

(22) Anmeldetag: **17.05.88**

(54) **Stoffaustauschsystem, insbesondere zur Befeuchtung von Gasen.**

(30) Priorität: **19.05.87 DE 3716653**

(43) Veröffentlichungstag der Anmeldung:
**23.11.88 Patentblatt  88/47**

(45) Bekanntmachung des Hinweises auf die
Patenterteilung:
**02.10.91 Patentblatt  91/40**

(84) Benannte Vertragsstaaten:
**DE FR GB SE**

(56) Entgegenhaltungen:
**GB-A- 2 053 693**
**GB-A- 2 053 694**
**GB-A- 2 053 695**
**GB-A- 2 082 921**
**US-A- 4 381 267**

(73) Patentinhaber: **Drägerwerk Aktiengesellschaft**
**Moislinger Allee 53-55**
**W-2400 Lübeck 1(DE)**

(72) Erfinder: **Sachtler, Jürgen**
**Lindenstrasse 38a**
**W-2400 Lübeck(DE)**
Erfinder: **Schmidt, Wolf-Dieter, Dipl.-Ing.**
**Blanckstrasse 24**
**W-2400 Lübeck(DE)**

**Beschreibung**

Die Erfindung betrifft ein Stoffaustauschsystem, insbesondere zur Befeuchtung von Gasen, mit mindestens einem Hohlfaserbündel, bei dem eine die Außenseiten der Hohlfasern umgebender erster Strömungsraum und ein durch die Innenräume der Hohlfasern gebildeter zweiter Strömungsraum vorhanden sind, wobei der Austausch zwischen den Strömungsräumen von den Austauschkennwerten der Hohlfasern abhängig ist sowie ein Verfahren zum Betrieb eines solchen Systems.

Ein Stoffaustauschsystem gemäß dem oberbegriff des Ausprruchs 1 ist aus der US-A-4 381 267 bekannt, zu deren Patentfamilie auch die EP-A-0 009 543 gehört.

Stoffaustauschsysteme, bei denen mindestens ein Hohlfaserbündel verwendet wird, werden in vielfacher Weise für den Stoffaustausch zwischen zwei Fluiden (Gas, Flüssigkeit) eingesetzt. Das eine Fluid strömt an der Außenseite der Hohlfasern in einen ersten Strömungsraum, während das andere Fluid in dem durch die Innenräume der Hohlfasern bestimmten zweiten Strömungsraum geführt wird. In der Praxis werden meist sogenannte Hohlfasermodule verwendet, welche jeweils ein Gehäuse aufweisen, in dem die Hohlfasern verlaufen, wobei der erste Strömungsraum zwischen der Innenwand des Gehäuses und den Außenseiten der Hohlfasern gebildet wird. Ein Stoffaustauschsystem im Sinne dieser Erfindung kann insbesondere aus einem oder mehreren derartiger Hohlfasermodule bestehen. Mehrere Module bilden ein System, wenn sie so miteinander zusammengeschaltet sind, daß sie insgesamt einem bestimmten Stoffaustauschzweck dienen.

Hohlfaser-Stoffaustauschsysteme werden für verschiedene Zwecke, beispielsweise im medizinischen Bereich als Dialysatoren oder Hämofilter eingesetzt. Ein weiteres Anwendungsgebiet bilden Luftbefeuchter, die beispielsweise in Verbindung mit Geräten zur künstlichen Beatmung verwendet werden. Derartige Atemluftbefeuchter sind in der DE-PS 26 17 985,in der EP-B1 0 009 543 sowie in der DE-OS 29 29 584 beschrieben. Die Erfindung richtet sich insbesondere auf solche Stoffaustauschsysteme für die Befeuchtung von Gasen, insbesondere von Atemluft.

Die Wände der Hohlfasern bestehen bevorzugt aus semipermeablen Membranen, die einen selektiven Übertritt von Substanzen aus dem einen Fluid in das andere ermöglichen. Bei der Atemluftbefeuchtung strömt Wasser durch den ersten Strömungsraum. Die Atemluft wird durch den zweiten Strömungsraum, also durch das Innere der Hohlfasern geleitet. Das Wasser wird üblicherweise erwärmt. Die Hohlfasern sind für Wassermoleküle durchlässig, so daß die durch ihren Innenraum strömende Atemluft befeuchtet wird. Der Grad der Befeuchtung ist von den Materialeigenschaften der Hohlfasern, insbesondere von ihrer Porengröße und von der Dimensionierung (Wandstärke, Durchmesser) der Fasern in dem Modul abhängig. Diese Einflußgrößen werden hier zusammengefaßt als Stoffaustauschkennwerte der Hohlfasern bezeichnet. Der Befeuchtungsgrad der Atemluft ist daneben auch von der Temperatur des durch den ersten Strömungsraum fließenden Wassers abhängig, weil dessen Dampfdruck mit steigender Temperatur zunimmt.

Bei der Atemluftbefeuchtung soll durch den Stoffaustauschmodul zugleich die Atemluft auf eine für den Patienten günstige Temperatur erwärmt werden. Der Grad der Erwärmung hängt vom Wärmeleitvermögen der Hohlfasern und von deren Dimensionierung (Wandstärke, Durchmesser) ab. Diese lassen sich als Wärmeaustauschkennwert der Hohlfasern zusammenfassen. Daneben hängt die Erwärmung der Atemluft selbstverständlich von der Temperatur des Wassers ab.

Wird die Temperatur des den ersten Strömungsraum durchfließenden Wassers verändert, so verändert sich jeweils sowohl die ausgetauschte Wärmemenge, als auch die ausgetauschte Wassermenge entsprechend den jeweiligen Austauschkennwerten. Infolgedessen ist es nicht möglich, den Feuchtigkeitsgehalt und die Temperatur der Atemluft unabhängig voneinander zu variieren. Dies wäre jedoch wünschenswert, weil aus medizinischer Sicht für verschiedene Patienten und für verschiedene Anwendungszwecke verschiedene - nach Möglichkeit im Betrieb des Systems veränderbare - Verhältnisse hinsichtlich Feuchtigkeit und Temperatur der Atemluft möglich sein sollten. Ähnliche Probleme bestehen auch auf anderen Gebieten, auf denen Stoffaustauschmodule eingesetzt werden und bei denen aufgrund der gegebenen Austauschkennwerte eine nicht gewünschte Kopplung des Austauschs verschiedener Stoffe und/oder Energie besteht.

Aufgabe der vorliegenden Erfindung ist es daher, ein Stoffaustauschsystem zur Verfügung zu stellen, bei dem der Austausch von Stoffen und von Energie flexibler als bisher eingestellt werden kann.

Diese Aufgabe wird bei einem Stoffaustauschsystem der eingangs bezeichneten Art dadurch gelöst, daß mindestens zwei Teilbereiche mit Hohlfasern unterschiedlicher Austauschkennwerte vorhanden sind.

Beispielsweise können in einem System Hohlfasern mit einem hohen Stoffaustauschvermögen, insbesondere einem sehr guten Wirkungsgrad für die Luftbefeuchtung mit anderen Hohlfasern kombiniert werden, die für Wasser weitgehend oder vollständig undurchlässig sind, jedoch spezielle Eigenschaften hinsichtlich ihrer Wärmeleitung haben. Dadurch lassen sich Gesamtaustauscheigenschaften erzielen, die mit einem System mit nur einem Hohlfasertyp nicht erreichbar sind.

Das erfindungsgemäße Stoffaustauschsystem kann im einfachsten Falle aus einer Mehrzahl von Stoffaustauschmodulen bestehen, die Hohlfasern unterschiedlicher Austauschkennwerte enthalten. Die die Außenseite der Hohlfasern umgebenden Räume sind durch Leitungen miteinander verbunden und werden von einem ersten Fluid, beispielsweise erwärmten Wasser, durchströmt, so daß sie insgesamt den ersten Strömungsraum bilden. Die Innenräume der Hohlfasern aller Stoffaustauschmodule werden von einem zweiten Fluid, insbesondere Luft, durchströmt und bilden insgesamt den zweiten Strömungsraum. Je nach Anwendungsfall können die Stoffaustauschmodule dabei hintereinander oder parallel geschaltet sein.

Gemäß einer bevorzugten Ausführungsform ist eine Strömungsumschaltung zwischen den Teilbereichen der Hohlfasern mittels eines Steuerelementes möglich. Für den Fall, daß das Stoffaustauschsystem aus einer Mehrzahl von Stoffaustauschmodulen besteht, kann als Steuerelement beispielsweise ein Mehrwegehahn in dem Leitungssystem vorgesehen sein. Je nach dessen Stellung wird beispielsweise die Atemluft in einem Atemluftbefeuchtungssystem auf die verschiedenen Stoffaustauschmodule verteilt und je nach deren Austauschkennwerten befeuchtet und erwärmt. Die Einstellung kann auch in Betrieb geändert werden.

Die Verteilung der Strömung auf die verschiedenen Teilbereich des zweiten Strömungsraumes mit Hilfe des Steuerelementes ist bevorzugt so ausgebildet, daß die einen Teilbereich durchströmende Fluidmenge abnimmt, während die einen anderen Teilbereich durchströmende Fluidmenge zunimmt. Bevorzugt bleibt dabei der Gesamtwiderstand der Strömung durch den zweiten Strömungsraum konstant.

Die Erfindung wird im folgenden anhand eines in den Figuren schematisch dargestellten Ausführungsbeispiels näher erläutert; es zeigen:

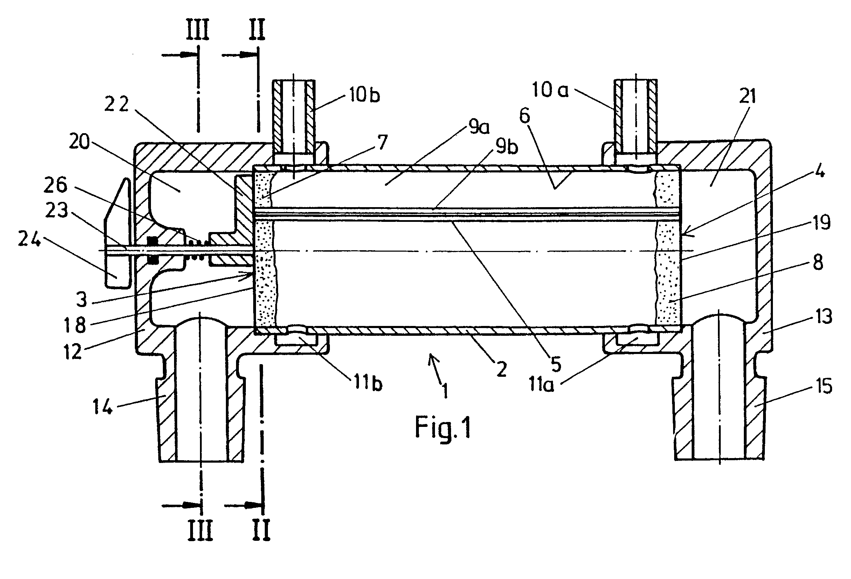

Fig. 1    einen Längsschnitt durch einen Stoffaustauschmodul zur Atemluftbefeuchtung,

Fig. 2    einen Querschnitt entlang der Linie II-II von Fig. 1,

Fig. 3    einen Querschnitt entlang der Linie III-III von Fig. 1,

Fig. 4    eine Draufsicht auf die Stirnseite des Moduls nach Fig. 1.

Die Figuren zeigen einen Stoffaustauschmodul 1, bei dem verschiedene Teilbereiche mit Hohlfasern unterschiedlicher Austauschkennwerte in einem einzigen Gehäuse 2 zusammengefaßt sind. Das Gehäuse 2 ist zylinderförmig und hat zwei kreisförmige Öffnungen 3 und 4. In dem Gehäuse verlaufen achsparallel eine Vielzahl von Hohlfasern 5, die in den Figuren stark vergrößert dargestellt sind. Die Enden der Hohlfasern 5 sind in Bereich der Gehäuseöffnungen 3 und 4 miteinander und mit der Innenwand 6 des Gehäuses flüssigkeitsdicht verbunden. Bei der dargestellten Ausführungsform ist dies mittels einer Vergußmasse 7,8 realisiert.

Der die Außenseite der Hohlfasern 5 umgebende erste Strömungsraum 9a, der nach außen durch das Gehäuse 2 begrenzt wird, ist über zwei Anschlußstutzen 10a, 10b, und Ringkanäle 11a, 11b an einen Kreislauf für ein erstes Fluid anschließbar.

Auf die Öffnungen 3,4 des Gehäuses 2 sind Endstücke 12,13 dichtend aufgesetzt. Die Endstücke 12,13 sind jeweils mit Anschlußstutzen 14,15 versehen. Durch die Anschlußstutzen 14,15 und die Endstücke 12,13 kann der durch die Innenräume der Hohlfasern 5 gebildete zweite Strömungsraum 9b an einen Kreislauf für ein zweites Fluid angeschlossen werden.

Im Falle des dargestellten Atemluftbefeuchters ist das erste Fluid erwärmtes Wasser, das zweite Fluid die Atemluft. Die, wie erwähnt, stark vergrößert dargestellten Hohlfasern bestehen bevorzugt aus Polysulfon und haben einen Innendurchmesser von etwa 1 - 3 mm und eine Wandstärke von 0,05 mm - 0,2 mm. Ein Hohlfasermodul enthält im allgemeinen mehrere hundert Hohlfasern. Soweit bisher beschrieben, ist der dargestellte Atemluftbefeuchter ähnlich dem in der oben zitierten europäischen Patentschrift 9 543 aufgebaut. Er kann auch in der dort beschriebenen Art und Weise hergestellt und verwendet werden.

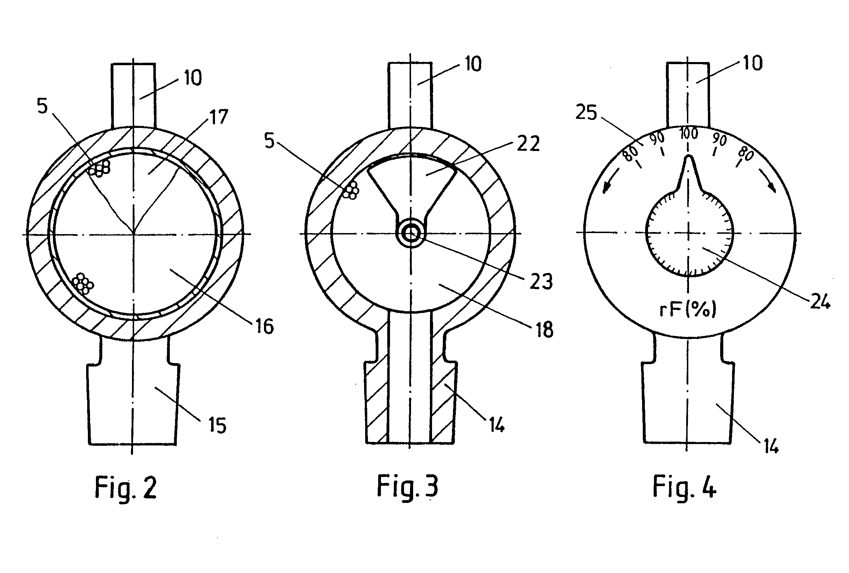

Die Hohlfasern in dem Gehäuse 2 sind nicht alle gleich, sondern sind bei der dargestellten Ausführungsform in zwei Teilbereiche 16 und 17 unterteilt, wobei sich die Hohlfasern der Teilbereiche hinsichtlich ihrer Austauschkennwerte voneinander unterscheiden. Wie aus Fig. 2 zu ersehen ist, sind die Teilbereiche 16,17 jeweils im Querschnitt des Gehäuses 2 betrachtet kreissektorförmig angeordnet.

Bei der Herstellung des Moduls werden nach dem Vergießen die Enden der Hohlfasern 5 zusammen mit der überstehenden Vergußmasse 7,8 glatt abgeschnitten. Dadurch wird an jeder Öffnung 3,4 des Gehäuses eine Eintrittsebene 18,19 gebildet, in der die Innenräume der Hohlfasern 5 in die Innenräume 20,21 der Endstücke 12,13 münden.

Auf einer der Eintrittsebenen 18 ist ein im wesentlichen kreissektorförmiges Absperrelement 22 um eine zum Gehäuse 2 koaxiale Achse 23 schwenkbar. Es läßt sich mit einem auf der Achse 23 befestigten Stellknopf 24 bewegen. In axialer Richtung ist es mit der Achse 23 verschiebbar und wird mittels einer Druckfeder 26 gegen die Eintrittsebene 18 gedrückt.

Im Betrieb wird die Atemluft über den Anschlußstutzen 14 zugeführt und durchströmt den durch die Innenräume der Hohlfasern gebildeten zweiten Strömungsraum 9b, über den Anschlußstutzen 15 gelangt sie zum Patienten. Gleichzeitig zirkuliert Wasser einer bestimmten Temperatur, welches durch den Anschlußstutzen 10a zugeführt und durch den Anschlußstutzen 10b abgeführt wird, durch den ersten Strömungsraum 9a. Durch das Absperrelement 22 wird ein Teil der Hohlfasereintrittsöffnungen in der Eintrittsebene 18 verschlossen. Bei der dargestellten Ausführungsform ist der von dem Absperrelement 22 abgesperrte Teilbereich der Hohlfasereintrittsöffnungen etwa ebenso groß wie der Teilbereich 17 der Hohlfasern, so daß das Absperrelement 22 in der in Fig. 3 dargestellten Stellung den Teilbereich 17 der Hohlfasern praktisch vollständig absperrt. Der Austausch erfolgt dann nur über den Teilbereich 16 der Hohlfasern. Demzufolge entspricht die von dem Wasser auf die Atemluft übertragene Menge an Wärme und Feuchtigkeit dem Austauschkennwerten der Hohlfasern in dem Teilbereich 16. Je mehr das Absperrelement aus der in Fig. 3 dargestellten Lage herausgeschwenkt wird, desto mehr werden die Öffnungen der Hohlfasern in dem Teilbereich 17 freigelegt und gleichzeitig ein Teil der Eintrittsöffnungen der Hohlfasern in dem Teilbereich 16 verschlossen. Dadurch werden die Eigenschaften des Gesamtmoduls in Richtung auf die Austauschkennwerte der Hohlfasern in dem Teilbereich 17 verändert.

Dies sei an einem Beispiel näher erläutert: Der Teilbereich 16 enthält Hohlfasern, deren Wärmeaustauschkennwert bei einer bestimmten Wassertemperatur $T_1$ eine gewünschte Erwärmung der Atemluft auf eine Temperatur $T_2$ sicherstellt und zugleich bei der gleichen Wassertemperatur zu einer Wasserdampfsättigung der Atemluft, also zu einer relativen Luftfeuchtigkeit von 100 % führt. Der Teilbereich 17 enthält Hohlfasern, welche sich hinsichtlich ihres Wärmeaustauschkennwertes nicht von denen im Teilbereich 16 unterscheiden, jedoch für Wasser undurchlässig sind. Bei einem derartigen Modul wird in der in Fig. 3 dargestellten Mittelstellung eine Luftfeuchtigkeit von 100 % bei gleichzeitiger Erwärmung der Atemluft auf $T_2$ erreicht. Durch Verstellen des Stellknopfes 24 und damit des Absperrelementes 22 wird ein Teil der wasserdurchlässigen Hohlfasern abgesperrt und zugleich ein entsprechender Teil der wasserundurchlässigen Hohlfasern freigelegt. Dadurch sinkt die Wasserübertragung im Gesamtmodul, und es wird eine reduzierte Luftbefeuchtung bei gleichbleibender Atemlufttemperatur $T_2$ erreicht. Das Endstück 12 kann außen mit einer Skala 25 versehen sein, wie dies in Fig. 4 dargestellt ist.

Bei der hier beschriebenen Ausführungsform der Erfindung ist es also möglich, den Gesamtaustausch im Modul bezüglich einer Austauschgröße (Befeuchtungsgrad) zu verändern, ohne daß gleichzeitig eine zweite für die Funktion des Moduls wesentliche Austauschgröße (Wärmeübertragung) in einer durch die Austauschkennwerte der verwendeten Hohlfasern zwangsläufig sich ergebenden Art und Weise mitverändert werden muß. Insbesondere ist es möglich, die Wärmeübertragung konstant zu halten und gleichzeitig die Befeuchtung zu variieren. Diese Veränderung kann während des Betriebs des Luftbefeuchters vorgenommen werden.

Soweit der Strömungswiderstand pro Flächeneinheit der Eintrittsebene 18 in den Teilbereichen 16 und 17 gleich ist, ist der Strömungswiderstand der Atemluft unabhängig von der Einstellung des Absperrelementes 22.

In einigen Anwendungsfällen ist es darüberhinaus wünschenswert, daß innerhalb bestimmter Grenzen sowohl die Atemlufttemperatur als auch ihre Befeuchtung völlig unabhängig voneinander wählbar sind. Hierzu sind mindestens drei Teilbereiche von Hohlfasern mit verschiedenen Austauschkennwerten erforderlich. Die Funktion eines derartigen Moduls läßt sich am besten anhand eines weiteren Beispiels erläutern:

Will man eine Einstellbarkeit der Temperatur im Bereich zwischen 35 und 40° C und eine Befeuchtung der Atemluft zwischen 80 und 100% relative Luftfeuchtigkeit ermöglichen, so werden drei verschiedene Teilbereiche A,B,C von Hohlfasern verwendet, die jeweils für sich genommen (also, wenn 100 % der Atemluft durch den jeweiligen Teilbereich strömen) bei einer vorgegebenen Wassertemperatur $T_0$ zu folgendem Ergebnis hinsichtlich der Temperatur und Befeuchtung der Atemluft führen:

A: 40° C, 100 % rF (relative Luftfeuchtigkeit)
B: 40° C, 0 % rF
C: 18° C, 0 % rf

Auf dieser Basis lassen sich die Eckpunkte der gewünschten Bereiche realisieren, indem man die Atemluft zu den in der folgenden Tabelle angegebenen Prozentsätzen durch die einzelnen Teilbereiche der Hohlfasern strömen läßt:

100 % A : 40° C, 100 % rF
25,6 % C + 74,4 % A : 35° C, 100 % rF
21,6 % B + 78,4 % A : 40° C, 80 % rF
58,6 % A + 18,6 % B + 22,8 % C : 35° C, 80 % rF

Das gleiche Ergebnis läßt sich auch mit anderen Werten der drei Teilbereiche der Hohlfasern erreichen, welche sich empirisch oder mit Hilfe des Mollier-h-x-Diagramms für feuchte Luft ermitteln lassen.

Die Arbeitsweise des Gesamtsystems ist im wesentlichen unabhängig von der im Beispiel angegebenen Strömungsrichtung der Fluide.

**Patentansprüche**

1. Stoffaustauschsystem, insbesondere zur Befeuchtung von Gasen, mit mindestens einem Hohlfaserbündel, bei dem ein die Außenseiten der Hohlfasern umgebender erster Strömungsraum und ein durch die Innenräume der Hohlfasern gebildeter zweiter Strömungsraum vorhanden sind, wobei der Austausch zwischen den Strömungsräumen von den Austauschkennwerten der Hohlfasern abhängig ist, **dadurch gekennzeichnet, daß** mindestens zwei Teilbereiche (16,17) mit Hohlfasern unterschiedlicher Austauschkennwerte vorhanden sind.

2. Stoffaustauschsystem nach Anspruch 1, **dadurch gekennzeichnet, daß** der erste Strömungsraum (9a) zur Aufnahme einer Flüssigkeit, insbesondere Wasser, und der zweite Strömungsraum (9b) zur Aufnahme eines Gases, insbesondere Luft, ausgebildet ist.

3. Stoffaustauschsystem nach Anspruch 2, **dadurch gekennzeichnet, daß** die Austauschkennwerte der Hohlfasern in den Teilbereichen (16,17) sich bezüglich ihrer Durchlässigkeit für die Flüssigkeit in Relation zu ihrer Wärmeleitfähigkeit unterscheiden.

4. Stoffaustauschsystem nach Anspruch 1, **dadurch gekennzeichnet, daß** mindestens ein Steuerelement (22) zur Strömungsumschaltung zwischen den Teilbereichen (16,17) der Hohlfasern vorgesehen ist.

5. Stoffaustauschsystem nach Anspruch 4, **dadurch gekennzeichnet, daß** das Steuerelement (22) so ausgebildet ist, daß bei der Strömungsumschaltung die Strömung durch einen Teilbereich (16) abnimmt, während die Strömung durch einen anderen Teilbereich (17) zunimmt.

6. Stoffaustauschsystem nach Anspruch 5, **dadurch gekennzeichnet, daß** das Steuerelement (22) so ausgebildet ist, daß bei der Strömungsumschaltung der Gesamtwiderstand der Strömung durch den zweiten Strömungsraum (9b) näherungsweise konstant bleibt.

7. Stoffaustauschsystem nach Anspruch 4, **dadurch gekennzeichnet, daß** die Hohlfasern in einem Gehäuse zu einem Hohlfasermodul zusammengefaßt sind, wobei die Enden der Hohlfasern miteinander und mit der Innenwand des Gehäuses im Bereich einer Gehäuseöffnung unter Bildung einer Eintrittsebene in die Innenräume der Hohlfasern verbunden sind, und daß als Steuerelement ein auf der Eintrittsebene (18) bewegliches Absperrelement (22) vorgesehen ist.

8. Stoffaustauschsystem nach Anspruch 7, **dadurch gekennzeichnet, daß** die Eintrittsebene (18) kreisförmig ist, die Hohlfasereintrittsöffnungen der Teilbereiche (16,17) kreissektorförmig in der Eintrittsebene angeordnet sind, und daß das Absperrelement (22) kreissektorförmig ausgebildet und um eine senkrecht zum Kreismittelpunkt verlaufende Achse (23) schwenkbar ist.

9. Stoffaustauschsystem nach Anspruch 3, **dadurch gekennzeichnet, daß** mindestens drei Teilbereiche der Hohlfasern vorhanden sind.

10. Verfahren zum Betrieb eines Stoffaustauschsystems nach Anspruch 1, **dadurch gekennzeichnet, daß** ein erstes Fluid durch den ersten Strömungsraum strömt und ein zweites Fluid durch den zweiten Strömungsraum strömt, und daß der Gesamtstrom des zweiten Fluids in mindestens zwei Teilströme aufgeteilt wird, die durch die Teilbereich der Hohlfasern strömen.

11. Verfahren nach Anspruch 10, **dadurch gekennzeichnet, daß** das Teilungsverhältnis zwischen den Teilströmen im Betrieb verändert wird.

**Claims**

1. A material transfer system in particular for humidifying gases, with at least one hollow fibre bundle, in which there are provided a first flow chamber surrounding the outer sides of the hollow fibres and a second flow chamber, formed by the inner chambers of the hollow fibres, wherein the transfer between the flow chambers is dependent on the transfer characteristic values of the hollow fibres, characterised in that at least two partial regions (16, 17) are provided with hollow fibres of differing transfer characteristic values.

2. A material transfer system according to claim 1, characterised in that the first flow chamber (9a) is designed for accepting a liquid, in particular water, and the second flow chamber (9b) for accepting a gas, in particular air.

3. A material transfer system according to claim 2, characterised in that the transfer characteristic values of the hollow fibres in the partial regions (16, 17) differ regarding their permeability to the liquid in relation to their heat conductivity.

4. A material transfer system according to claim 1, characterised in that at least one control element (22) is provided for flow change-over between the partial regions (16, 17) of the hollow fibres.

5. A material transfer system according to claim 4, characterised in that the control element (22) is formed so that with the flow change-over the flow is reduced through one partial region (16), whilst the flow increases through another partial region (17).

6. A material transfer system according to claim 5, characterised in that the control element (22) is formed so that with flow change-over the total resistance of the flow through the second flow chamber (9b) remains approximately constant.

7. A material transfer system according to claim 4, characterised in that the hollow fibres in a housing are combined in a hollow fibre module, wherein the ends of the hollow fibres are connected to each other and to the inner wall of the housing in the region of a housing opening with formation of an inlet plane into the inner chambers of the hollow fibres, and in that as control element there is provided a blocking element (22) movable on the inlet plane (18).

8. A material transfer system according to claim 7, characterised in that the inlet plane (18) is circular, the hollow fibre inlet openings of the partial regions (16, 17) are arranged in a circular sector shape in the inlet plane and in that the blocking element (22) is formed in a circular sector shape and swivelled around an axis (23) extending perpendicular to the central point of the circle.

9. A material transfer system according to claim 3, characterised in that at least three partial regions of the hollow fibres are present.

10. A method for operating a material transfer system according to claim 1, characterised in that a first fluid flows through the first flow chamber and a second fluid through the second flow chamber, and in that the total flow of the second fluid is divided into at least two partial flows, which flow through the partial regions of the hollow fibres.

11. A method according to claim 10, characterised in that the dividing ratio between the partial flow is altered when in operation.

**Revendications**

1. Système d'échange de matières notamment pour l'humidification de gaz, comportant au moins un faisceau de fibres creuses, dans lequel il existe une première chambre d'écoulement, entourant les côtés extérieurs des fibres creuses et une deuxième chambre d'écoulement, formée par les cavités intérieures des fibres creuses, l'échange entre les chambres d'écoulement étant fonction des caractéristiques d'échange des fibres creuses, caractérisé en ce que deux zones partielles (16, 17) au moins présentent des fibres creuses dont les caractéristiques d'échange sont différentes.

2. Système d'échange de matières selon la revendication 1, caractérisé en ce que la première chambre d'écoulement (9a) est conçue pour recevoir un liquide, notamment de l'eau et la deuxième chambre d'écoulement (9b) pour recevoir un gaz, notamment de l'air.

3. Système d'échange de matières selon la revendication 2, caractérisé en ce que les caractéristiques d'échange des fibres creuses dans les zones partielles (16, 17) diffèrent par leur perméabilité au liquide, par rapport à leur conductibilité thermique.

4. Système d'échange de matières selon la revendication 1, caractérisé en ce qu'il est prévu au moins un élément de commande (22) pour commander le changement d'écoulement entre les zones partielles (16, 17) des fibres creuses.

5. Système d'échange de matières selon la revendication 4, caractérisé en ce que l'élément de commande (22) est conçu de manière que, lors du changement d'écoulement, l'écoulement à travers une zone partielle (16) diminue, tandis qu'il augmente à travers une autre zone partielle (17).

6. Système d'échange de matières selon la revendication 5, caractérisé en ce que l'élément de commande (22) est conçu de manière que, lors du changement d'écoulement, la résistance totale à l'écoulement à travers la deuxième chambre d'écoulement (9b), reste à peu près constante.

7. Système d'échange de matières selon la revendication 4, caractérisé en ce que les fibres creuses sont réunies dans une enveloppe pour former un module de fibres creuses, les extrémités des fibres creuses étant reliées entre elles et à la paroi intérieure de l'enveloppe dans la région d'une ouverture d'enveloppe, par formation d'un plan d'entrée dans les cavités intérieures des fibres creuses et en ce qu'il est prévu comme élément de commande, un élément d'arrêt (22) mobile sur le plan d'entrée (18).

8. Système d'échange de matières selon la revendication 7, caractérisé en ce que le plan d'entrée (18) est circulaire, les ouvertures d'entrée de fibres creuses des zones partielles (16, 17) sont placées en forme de secteurs de cercle dans le plan d'entrée et en ce que l'élément d'arrêt (22) a la forme d'un secteur de cercle et peut pivoter autour d'un axe (23) perpendiculaire au centre du cercle.

9. Système d'échange de matières selon la revendication 3, caractérisé en ce qu'il est prévu au moins trois zones partielles des fibres creuses.

10. Procédé pour l'exploitation d'un système d'échange de matières selon la revendication 1, caractérisé en ce qu'un premier fluide s'écoule à travers la première chambre d'écoulement et un deuxième fluide à travers la deuxième chambre d'écoulement et en ce que le courant total du deuxième fluide est partagé en au moins deux courants partiels qui s'écoulent à travers les zones partielles des fibres creuses.

11. Procédé selon la revendication 10, caractérisé en ce que le rapport de partage entre les courants partiels est modifié pendant le fonctionnement.

Fig.1

EP 0 291 921 B1

Fig. 2

Fig. 3

Fig. 4